# EUROPEAN PATENT SPECIFICATION

(11) **EP 3 941 553 B1**
(45) Date of publication and mention of the grant of the patent: **22.07.2026**
(21) Application number: 20710195.7
(22) Date of filing: 17.03.2020
(51) Int. Cl.: A61M 5/24

(54) **CONTAINER RETAINING MEMBER, DRUG DELIVERY DEVICE AND METHOD**
BEHÄLTERHALTEELEMENT, ARZNEIMITTELABGABEVORRICHTUNG UND -VERFAHREN
ÉLÉMENT DE RETENUE D'UN RÉCIPIENT, DISPOSITIF D'ADMINISTRATION DE MÉDICAMENTS ET PROCÉDÉ

(30) Priority: 20.03.2019 EP 19305339
(43) Date of publication of application: 26.01.2022
(73) Proprietor: SANOFI, 75017 Paris (FR)
(72) Inventor: HINTERMEIER, Phillip, 65926 Frankfurt am Main (DE)
(74) Representative: Schmidt, Christian
(86) International application number: PCT/EP2020/057198
(87) International publication number: WO 2020/187876

(56) References cited:
- US-A1- 2003 004 466
- US-A1- 2014 343 503
- US-A1- 2018 318 509

## Description

The disclosure relates to a container retaining member, preferably for a drug delivery device, and to a drug delivery device that comprises the container retaining member and/or a corresponding assembly. Furthermore, a method for operating a container retaining member is disclosed. US 2003/004466 A1 discloses a container retaining member according to the state of the art.

It is an object of the disclosure to provide an improved container retaining member, especially a container retaining member that hinders or prevents manipulations and/or that makes sure that only the correct containers are used, preferably in connection with containers that comprise drugs or medicaments. Furthermore, a corresponding device and method shall be disclosed.

This object may be achieved by the container retaining member according to claim 1 and by a drug delivery device and a method according to the independent claims. Further advantageous embodiments are given in the dependent claims.

According to the invention, the container retaining member is a container retaining member for a drug delivery device. The container retaining member comprises:
- a retaining member body comprising an inner surface that limits or surrounds a retaining space for a container, wherein the retaining member body has an insertion opening through which the container is insertable into the retaining space, and
- a fixing member comprising:
- a connecting portion that is connected to the inner surface and extends inwardly from the inner surface of the retaining member body,
- a first fixing portion that extends from the connecting portion into the retaining space and that comprises a first free end,
- a second fixing portion that extends from the connecting portion and/or from the first fixing portion into the retaining space and that comprises a second free end,

wherein the first fixing portion and the second fixing portion are arranged and configured to engage the container in order to secure the container within the container retaining member, and
wherein the first free end is arranged closer to the insertion opening than the second free end.

It is difficult to manipulate the fixing member because the connecting portion extends inwardly from the inner surface of the retaining member body, i.e. the whole fixing member may be surrounded by the retaining member body. Thus, it is possible to insert a container during production containing the right amount and the right concentration of a drug. The patient may insert the container retaining member into a pen type device and may inject the drug at one time or at several times. When the container is empty the patient may replace the whole retaining member body by a new one that comprises again the correct drug, the correct amount and the correct concentration. The container retaining member may be coded, for instance mechanically, to fit only to a special drug delivery device in order to make sure that the patient gets the correct drug and/or the correct concentration of the drug.

The first free end and the second free end may be arranged to prevent each other from losing contact to the container, especially when all fixing members are considered. Therefore, the container is fixed in the distal direction and in the proximal direction. The distal end may be close to an injection end of the container retaining member. The proximal end may be closer to the insertion opening.

In the case in which the second fixing portion extends from the connecting portion into the retaining space there may be a bifurcation from which the first fixing portion and the second fixing portion extend into the retaining space in different directions. Both fixing portions may have the same shape or a similar shape in this case. The fixing member having this bifurcation may be a very stable fixing member that is able to withstand a higher pressure force and/or pulling force.

In the case in which the second fixing portion extends from the first fixing portion into the retaining space the first free end may be arranged on a protrusion of the first fixing portion. The connecting portion, the first fixing member and the second fixing portion may be arranged in this sequence. This sequence may allow a comparably flat arrangement that may be appropriate to have a small installation space.

The first fixing portion and the second fixing portion may abut against the container. It is possible that the first free end and/or the second free end also abut(s) against the container.

The first free end and/or the second free end may have the shape of longer edges, for instance in the range of 1 mm to 5 mm (millimeter). Alternatively, the free ends may have a rounder shape, especially if finger shaped fixing portions are used. The first free end may be arranged on a protrusion of the first fixing portion in the case in which the second fixing portion is connected to the first fixing portion, especially in the case in which the second fixing portion is only connected to the first fixing portion but not to the connecting portion directly.

The container and the container retaining member may each be elongated. The length of the container and/or the container retaining member may be for instance at least three, four or five times the maximum width of the container and for instance at most 20 times the maximum width of the container.

The container may comprise a container main body and a container cover. The container main body may for instance be made of glass or may comprise glass. The container cover may be for instance made of metal or comprise metal. The container cover may be fastened to the container main body for instance by border crimping.

The drug container may be, e.g., a cartridge, syringe, reservoir, ampoule, or other solid or flexible vessel configured to provide a suitable chamber for storage (e.g., short- or long-term storage) of one or more drugs. The syringe may already comprise a needle that is used for injection.

There may be a non-deflected position, for instance a first state, of the fixing member when no container is inserted into the container retaining member. Furthermore, there may be a deflected position, for instance a second state, of the fixing member when the container is inserted. The deflection may generate a force for holding the container. Further deflection may arise at one of the fixing portions or on both fixing portions. The further deflection may add a further force for holding the container.

The connecting portion is movably connected to the inner surface of the retaining member body. The fixing member is configured such that for switching between a first state and a second state the first free end and/or the second free end move(s) axially relative to an insertion axis that is arranged crosswise or perpendicular to the insertion opening. Perpendicular may refer to a right angle, i.e. an angle of 90 degrees. The axial movement is may possible because the fixing member pivots around a fixing position on the retaining member body. The insertion opening is widened or expanded by this movement in order to allow a cap of the container to pass. The insertion opening may contract thereafter to a neck portion of the container that has a smaller diameter or width than the cap of the container. Additionally, it may be possible to arrange the second free end by this movement between the inner surface and the container, especially the container cap. The first free end and/or the second free end may preferably be displaceable by at least 1 mm (millimeter), by at least 2 mm, by at least 4 mm or by at least 5 mm. The displacement may be smaller than 1.5 cm (centimeter) or smaller than 1 cm.

Alternatively or additionally, the fixing member may be configured such that for the switching between the first state and the second state the first free end and/or the second free end move(s) away from the insertion opening through which the container is inserted into the container retaining member. This distal movement is possible because the fixing member pivots around a fixing portion on the fixing member body.

The fixing member may be configured such that in the second state the second fixing portion extends axially relative to the insertion axis and/or such that in the second state the second fixing portion is arranged at a radial position relative to the insertion axis, wherein this radial position is radially inwardly offset from the inner surface of the retaining member body. A more distal and/or a more inward position within the retaining member body may hamper manipulations.

The fixing member may be configured such that in the second state the second fixing portion is arranged between the container and the inner surface of the retaining member body. Thus, the fixing portion is sheltered on several sides or on all sides for instance by the cap of the container, by the retaining member body and/or by the main part of the container.

There may be a free space or clearance between the second fixing portion and the inner surface in the second state in which the container is inserted. This free space may effect that the lateral pressure or the radial pressure on the container is within an acceptable range.

Alternatively, the second fixing portion may be adjacent to and in contact with the inner surface, e.g. with no space left between the second fixing portion and the inner surface. This may allow higher clamping forces for fixing the container within the retaining member body.

A flexible portion may be arranged between the inner surface of the retaining member body and the connecting portion. The flexible portion may allow a movement, especially pivoting, of the fixing member. The usage of the flexible portion is simple and cost effective.

The flexible portion may comprise a film hinge that is formed by a thin film that is thinner than the parts that are connected to the film hinge. The film hinge may be made of plastics or of another appropriate material. The film hinge may have a thickness that is smaller than 1 millimeter or smaller than 0.1 millimeter. The thickness of the film hinge may be for instance greater than 0.01 millimeter. A deflection angle or a pivoting angle of the film hinge between the first state and the second state may be in the range of 20 degrees to 110 degrees or in the range of 30 degrees to 60 degrees.

Alternatively or additionally to the film hinge, at least one lateral flexible connection may be arranged between the first connecting portion and a second connecting portion of a second fixing member. The flexible portions, for instance the film hinges, may be enforced by the flexible connections, especially in a lateral or circumferential direction. This enforcement may be very important for increasing the pulling force so that the container retaining member can withstand if someone tries to remove the container from the retaining member body.

The flexible connection may be outwardly offset relative to the first fixing member and/or relative to the second fixing member. The flexible connection may extend in a circumferential direction of the insertion opening. Preferably, the at least one flexible connection may deflect by the same amount as the at least two of the fixing members that are in the neighborhood of the at least one flexible connection or by a less amount than these fixing members.

According to a first alternative, the retaining member body may be an elongated retaining member body. The fixing member may be formed unitarily or integrally with the elongated retaining member body. The length of the elongated retaining member body may be at least twice the width or at least three times longer than the maximum width of the elongated retaining member body and/or shorter than 20 times the width of the elongated retaining member body. Unitarily or integrally may mean that the same mold and especially the same cavity has been used to produce only one part in the same molding process. Alternatively or additionally, unitarily or integrally may mean that it is not possible to separate one element from the other without destroying one of these elements or both elements. Thus, a very durable and strong connection between the elongated retaining member body and the fixing member can be reached. Furthermore, the number of parts may be reduced resulting in a simpler parts logistics and in less costs being involved. The elongated retaining member body may cover at least 50 percent, at least 75 percent or at least 90 percent of the length of the container. The elongated retaining member body may cover the length of the container completely. Thus, the elongated retaining member may extend to the proximal end of the container or it may extend beyond the proximal end.

Still referring to the first alternative, however, the fixing member may be formed on a separate part that is connected to the elongated retaining member body after the production of these two parts. Between these parts, there may be a mechanical connection, for instance by snap fit, tight fit/force fit, and/or a connection that uses a chemical adhesive, i.e. glue. This means that the compact retaining member body may be mounted into an elongated container holder.

According to a second alternative, the retaining member body may be a short or compact body that may have a length that is shorter than the width of the retaining member body or shorter than the maximum outer diameter of the retaining member body. Also in this case the fixing member may be formed unitarily with the retaining member body. The short retaining member body may ease the production of the container retaining member considerably because a mold may be made simpler, for instance using less sliders, i.e. sliding parts that are different from ejectors, compared to the case that involves an elongated retaining member body. With regard to the second alternative two separate parts may be used to accommodate the container within the retaining space. Between these parts, there may be a mechanical connection, for instance by snap fit, tight fit/force fit, and/or a connection that uses a chemical adhesive, i.e. glue. This means that the compact retaining member body may be mounted into an elongated container holder.

In both alternatives the container retaining member and/or the retaining member body may be made of plastics or of another appropriate material. Furthermore, in both alternatives the maximal outer diameter of the container may be within the range of 5 mm (millimeter) to 15 mm or within the range of 7 mm (millimeter) to 12 mm. Thus, the maximal inner diameter of the retaining member body may be slightly greater, for instance within the range of 6 mm to 16 mm or within the range of 8 mm to 13 mm
The first fixing portion may comprise a first surface that faces in the second state axially and/or distally relative to the insertion axis. The first surface may be planar or curved, spherical, aspherical, etc. A surface normal may be defined as the direction that faces away from the surface and that includes at all sides an angle of 90 degrees with the surface, especially with a planar surface. In the following, it is referred to the smaller angle of the two angles that are formed between the surface normal and an axis if nothing else is said.

In the second state, the following may be valid, especially for the first planar surface:
a) an angle between a surface normal of the first planar surface and the insertion axis is smaller than 10 degrees or smaller than 5 degrees with the surface normal of the first planar surface pointing to the insertion axis, or
b) a surface normal of the first planar surface and the insertion axis are parallel relative to each other, or
c) an angle between a surface normal of the first planar surface of the fixing member and the insertion axis is smaller than 45 degrees with the surface normal of the first planar surface of the fixing member pointing away from the insertion axis.

The second fixing portion may comprise a second surface that in the second state faces radially and/or inwardly relative to the insertion axis. The second surface may be planar or curved, spherical, aspherical, etc. A surface normal may be defined as the direction that faces away from the surface and that includes at all sides an angle of 90 degrees with the surface. In the following, it is referred to the smaller angle of two angles that are formed between the surface normal and an axis if nothing else is said.

In the second state, the following may be valid, especially for the second planar surface:
a) a surface normal of the second planar surface and the insertion axis are at right angle relative to each other, or
b) an angle between a surface normal of the second planar surface and the insertion axis is greater than 80 degrees or greater than 85 degrees but smaller than 90 degrees.

The first surface and the second surface may include an acute angle, preferably an angle that is in the range of 30 degrees to 85 degrees or in the range of 45 degrees to 75 degrees. The technical effect of this acute angle may be that the angle is extended by the container, i.e. the first fixing portion and/or the second fixing portion, especially the first end and/or the second end, are deflected away from each other by the container. This deflection may generate an abutting force that holds the container stronger between the two surfaces than without such deflection.

The first fixing portion may comprise a third surface that is configured to guide the container during the insertion of the container into the retaining space. Furthermore, the third surface may in the second state face into a direction that is different from the direction in which the first surface faces in the second state and/or that is different from the direction in which the second surface faces in the second state. Especially in the second state, the third surface of the fixing member may be located closer to the insertion opening than the first surface. The third surface may be planar or curved, spherical, aspherical, etc. A surface normal may be defined as the direction that faces away from the surface and that includes at all sides an angle of 90 degrees with the surface. In the following, it is referred to the smaller angle of the two angles that are formed between the surface normal and an axis if nothing else is said.

In the second state, the following may be valid, especially for the third planar surface:
a) an angle between a surface normal of the third planar surface and the insertion axis is in the range of 45 degrees to 75 degree with the surface normal of the third surface pointing to the insertion axis.

The third surface of the fixing member, the first surface of the fixing member and the second surface of the fixing member may form in this order a step, preferably a hook, especially if the angles given above are used for aligning these surfaces with regard to the insertion axis.

The at least one fixing member may be a first fixing member and the container retaining member may comprise two fixing members, three fixing members or at least four fixing members. There may be less than 100 fixing members or fewer than 50 fixing members or fewer than 20 fixing members. The mold may be simpler when fewer fixing members are used. The clamping force that interacts with the container may be higher the more fixing members are used.

All fixing members may be formed like the first fixing member in order to have the same clamping forces. The fixing members may be equidistantly disposed in the circumferential direction in order to distribute the clamping forces equally around the container or more specifically around a container cap and/or a container neck.

The at least one fixing member may be formed as a plate or as a finger. In the case of a plate, the width of the fixing member may be larger than the length of the fixing member. The plate shape may result in a compact and/or rigid fixing member. In the case of a finger, the length of the fixing member may be larger than the width of the fixing member especially larger than twice the width.

The plate may have a trapezoid foot print. The first surface of the at least one fixing member and/or the third surface of the at least one fixing member may be formed on a protrusion of the at least one fixing member. The protrusion may preferably be spaced apart from an edge of the fixing member, preferably spaced apart by at least 0.5 mm or by at least 1 mm. The protrusion may also have a trapezoid foot print. The protrusion may have the same shape of the foot print as the fixing member and/or preferably a smaller foot print. This may ease manufacturing and/or molding of the container retaining member.

The container retaining member may comprise at least one distal stop element that limits the retaining space for the container in an axial direction. The distal stop element may comprise a through hole in its center, preferably a circular through hole. A needle or a nozzle may be inserted through the through hole and into the container.

In the second state the proximal movement of the container may mainly be prevented by the at least one fixing member. This may be valid in embodiments with a distal stop element or in embodiments without a further distal stop element, e.g. that is provided in addition to the at least one fixing member. One effect of the at least one fixing member is the prevention of a proximal movement of the container. However, the at least one fixing member may additionally also prevent a movement in the distal direction if the container is inserted to its end position or usage position.

The retaining space may have a first portion between the stop element and a first side of the fixing member and a second portion at the other side of the fixing member. The second portion may comprise at least 75 percent of volume or at least 80 percent of volume of the overall retaining space. Thus the container may abut against the stop element with a distal surface and the fixing member may be placed deep within the retaining space. Thus access to the fixing member from the insertion opening is almost impossible, especially when the container is already inserted.

Alternatively, no distal stopping element may be used. In the second state, a distal movement of the container may then mainly be prevented by the at least one fixing member. This means that the container is fixed in the distal and in the proximal direction mainly or only by the at least one fixing member.

A container retaining assembly may comprise a container retaining member as described above and a container that are preferably connected permanently with each other. Permanently may mean that the container may not be removed from the container retaining member without destroying either the container or the alt least one fixing member. The container may comprise a drug. The container may be connected to the container retaining member wherein the fixing member is in the second state, i.e. in the state that is different from a first state in which no container is connected to the container retaining member. The drug may comprise insulin, hormones, antibodies, one of the drugs listed below in this document or another drug.

According to an embodiment a) the container retaining member may have the following features:
- wherein the second fixing portion is directly connected to the first fixing portion but not directly connected to the connecting portion and wherein the first fixing portion is configured to be deformed elastically into a lateral space during an insertion of the container, especially by a cap of the container, and wherein the lateral space is bounded by the first fixing portion and/or preferably by the connection portion and by the retaining member body.

Thus, one, some arbitrarily selected or all of the following features may be realized in the embodiment a):
- the container may be inserted from the rear or proximal end of the container retaining member, and/or
- the fixing member may have elasticity and/or resiliency in relation to the container retaining member, and/or
- the first fixing portion may be oriented obliquely relative to the longitudinal axis of the container retaining member and/or a third surface of the first fixing portion may be an inclined surface (e.g. inclined with regard to a longitudinal axis of the container retaining member). The inclined surface may face proximally in the first state and/or in the second state, and/or
- the first fixing portion, preferably also the third surface and/or a first surface of the first fixing portion, may be deformed, preferably elastically, when a cap (e.g. a ferrule or a metal cap, for instance a crimped cap) of the container passes the first fixing portion during insertion of the container into the container retaining member, and/or
- the fixing member may have a front part (distal part) which forms a larger inner diameter of the insertion opening in the second state compared to an inner diameter of a more proximal part of the insertion opening in the second state in order to engage the cap of the container in its final inserted position, and/or
- the first fixing portion and/or the third surface and/or the first surface of the first fixing portion may engage into a neck portion of the container, e.g. the neck portion having a smaller outer diameter compared to a main portion of the container and/or compared to an outer diameter of a cap of the container, when the container is in its final inserted position, i.e. in the second state of the fixing member.

According to an embodiment b) the container retaining member may have the following features:
- wherein the first fixing portion is directly connected to the connecting portion and the second fixing portion is directly connected to the connecting portion and wherein there is a bifurcation from which the first fixing portion and the second fixing portion extend into the retaining space in the second state and wherein the first free end and the second free end are arranged to prevent each other from losing contact to the container in the second state, especially from losing contact to a cap of the container.

Thus, one, some arbitrarily selected or all of the following features may be realized in the embodiment b):
- the container may be inserted from the rear or proximal end of the container retaining member, and/or
- the fixing member may have elasticity or resiliency in relation to the container retaining member, and/or
- the first fixing portion may be oriented obliquely relative to the longitudinal axis of the container retaining member and/or a third surface of the first fixing portion may be an inclined surface (e.g. inclined with regard to a longitudinal axis of the container retaining member). The inclined surface may face proximally in the first state and/or in the second state. Alternatively, the fixing member may be tapered or may have a wedge shape, especially when seen in a cross section along the longitudinal axis of the container retaining member. The connection portion may be the basis from which the tapering starts, preferably into a distal direction, and/or
- optionally, the first fixing portion and/or the third surface of the first fixing portion may be deformed, preferably elastically, when a cap (e.g. a ferrule or a metal cap, for instance a crimped cap) of the container passes the first fixing portion and/or the third surface of the first fixing portion during insertion of the container into the container retaining member. Alternatively, there may be no deforming or no essential deforming of the first fixing portion and/or of the third surface of the first fixing portion when a cap (e.g. a ferrule or a metal cap, for instance a crimped cap) of the container passes the first fixing portion and/or the third surface of the first fixing portion during insertion of the container into the container retaining member, and/or
- the fixing member may have a front part (distal part) which forms a larger inner diameter of the insertion opening in the second state compared to an inner diameter of the insertion opening in a more proximal part of the fixing member in the second state in order to engage the cap of the container in its final inserted position, and/or
- the first fixing portion and/or the third surface and/or only a part of a first surface of the first fixing portion may engage into a neck portion of the container, e.g. the neck portion having a smaller outer diameter compared to a main portion of the container and/or compared to an outer diameter of a cap of the container, when the container is in its final inserted position, i.e. in the second state of the fixing member.

In both embodiments a) and b) resiliency may be achieved by:
- appropriate material thickness, and/or
- reduced wall thickness, and/or
- appropriate choice of material, e.g. plastic and/or metal, and/or
- providing one or more longitudinal slots.

It is possible to combine embodiments a) and b) with any one or with several other embodiments mentioned in this description, i.e. with embodiments that are mentioned in the first part before the list of Figures or in the description of the Figures.

A second aspect relates to a drug delivery device comprising the container retaining assembly mentioned above and a main housing part that is connected to the assembly. A drive mechanism may be retained in the main housing part wherein the drive mechanism is operable to dispense drug from the container. The drug delivery device may be a pen shaped or pen type device. Pen shaped may mean that a length of the drug delivery device may be in the range of 8 cm (centimeter) to 25 cm and/or a maximal outer diameter of the drug delivery device may be within the range of 5 mm (millimeter) to 15 mm.

Alternatively, the container retaining member or the container retaining assembly may form a holding device that is used to hold an ampoule, i.e. a container that does not comprise a movable piston or bung. The ampoule may be hold using the container retaining member during breaking off or for sawing off a glass portion of the ampoule that is used for closing the ampoule. Injuries may be prevented by using the container retaining member during this opening step or process of the ampoule. As opposed to an ampoule, a cartridge may comprise a movable piston or a movable bung. The piston or bung may be movable relative to a, preferably rigid, cartridge body of the cartridge, e.g. a glass body. The bung or piston may close and/or seal the cartridge body proximally. If the piston or bung is displaced distally relative to the cartridge body, liquid content, e.g. drug, may be dispensed from the interior of the cartridge body, particularly if fluid communication is established between the interior of the cartridge and the exterior. The interior of the cartridge body may be sealed distally by a pierceable septum, which may be pierced by a needle to establish fluid communication with the exterior. The septum may be retained at the cartridge body by a septum retainer, e.g. a, preferably crimped, cap such as a metal cap. The ampoule may be a unitary closed container which may have to be partly destroyed, e.g. by breaking off a section of the ampoule to get access to its interior. The container may be a primary container, e.g. a container that is in direct physical contact with the drug.

A further aspect relates to a method for using a container retaining member. The method may be performed using one of the container retaining members and/or with the container retaining assembly mentioned above. The method may comprise:
a) providing a container retaining member that comprises:
   - a retaining member body comprising an inner surface and an insertion opening, wherein the inner surface limits a retaining space for a container, and
   - a fixing member comprising:
      - a connecting portion that is connected to the inner surface,
      - a first fixing portion that is connected to the connecting portion and comprises a first free end,
      - a second fixing portion that is connected to the connecting portion and/or to the first fixing portion and comprises a second free end,
b) inserting the container through the insertion opening into the retaining space,
c) moving or pivoting at least a part of the fixing member or the whole fixing member when the container abuts the fixing member during the insertion of the container,
whereby at least a part of the fixing member or the whole fixing member is moved from a first state to a second state by moving the first free end and/or the second free end away from the insertion opening and axially relative to an insertion axis that is arranged crosswise or perpendicular to the insertion opening. In addition the free ends may also move radially outwardly relative to the insertion axis.

The first free end may be arranged on a protrusion of the first fixing portion if the second fixing portion is connected to the first fixing portion. The method may be used to produce the container retaining assembly mentioned above.

Thus, the features, advantages and technical effects that are valid for the container retaining member and its embodiments may also be valid for the assembly, the drug delivery device and for the method and vice versa.

The making and using of the presently preferred embodiments are discussed in detail below. It should be appreciated, however, that the present disclosure provides many applicable concepts that can be embodied in a wide variety of specific contexts. The specific embodiments discussed are merely illustrative of specific ways to make and use the disclosed concepts, and do not limit the scope of the claims.

Moreover, same reference signs refer to same technical features if not stated otherwise. As far as "may" is used in this application it means the possibility of doing so as well as the actual technical implementation. The present concepts of the present disclosure will be described with respect to preferred embodiments below in a more specific context namely a drug delivery device. The disclosed concepts may also be applied, however, to other situations and/or arrangements as well.

The foregoing has outlined rather broadly the features and technical advantages of embodiments of the present disclosure. Additional features and advantages of embodiments of the present disclosure will be described hereinafter, e.g. of the subject-matter of dependent claims. It should be appreciated by those skilled in the art that the conception and specific embodiments disclosed may be readily utilized as a basis for modifying or designing other structures or processes for realizing concepts which have the same or similar purposes as the concepts specifically discussed herein. It should also be recognized by those skilled in the art that equivalent constructions do not depart from the scope of the disclosure, such as defined in the appended claims.

For a more complete understanding of the presently disclosed concepts and the advantages thereof, reference is now made to the following description in conjunction with the accompanying drawings. The drawings are not drawn to scale. In the drawings the following is shown in:
- Figure 1: a first embodiment of a container retaining member in a first state,
- Figure 2: the container retaining member of Figure 1 in a second state,
- Figures 3A and 3B: cross sections of a second embodiment,
- Figures 4A, 4B and 4C: cross sections of a third embodiment, and
- Figure 5: a drug delivery device.

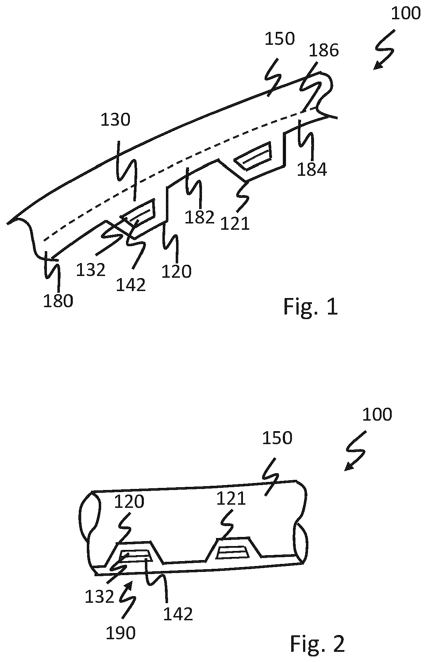

Figure 1 illustrates a first embodiment of a container retaining member 100 in a first state in which no container is inserted. Container retaining member 100 may have the shape of a closed ring. Alternatively, other shapes are possible as described below.

Two fixing members 120, 121 are shown in Figure 1. However, there are more than two fixing members 120, 121 arranged on a retaining member body 150 of the container retaining member 100. The first fixing member 120 comprises:
- a connecting portion 130 that is connected to retaining member body 150,
- a first fixing portion 132 that is connected to the connecting portion 130, and
- a second fixing portion 142 that is connected to the first fixing portion 132 and/or to connecting portion 130.

Alternatively both fixing portions 132 and 142 may only be directly connected to connecting portion 130.

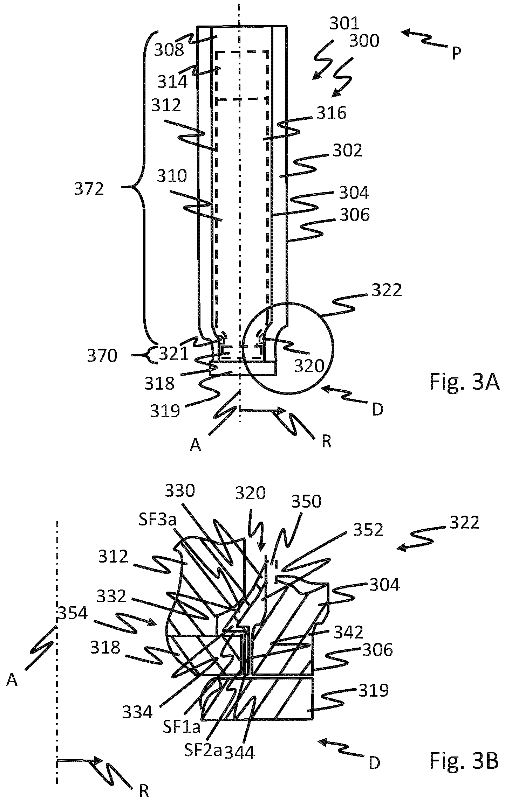

First fixing portion 132 may comprise a first free end. Second fixing portion 142 may comprise a second free end. The container may be hold between first fixing portion 132 and second fixing portion 142 or preferably between the first free end and the second free end. Examples for the detailed design of the fixing portions are shown in Figures 3A to 4C. Other designs are possible as well.

Second fixing member 121 may have the same shape as the first fixing member. Both fixing members 120 and 121 may extend axially inwardly in the state that is shown in Figure 1. This may be a first state, for instance the state after container retaining member 100 is taken out of a mold for injection molding.

Optionally, there may be flexible connections 180, 182, 184 between the fixing members 120, 121 etc. The flexible connections 180, 182, 184 may be an integral part of container retaining member 100. Flexible connections 180, 182, 184 are set more radially outward than the fixing members 120 and 121. Thus, flexible connections 180, 182, 184 may form a thin belt. Free spaces or clearances are left between the fixing members 120, 121. The fixing members 120, 121 may have a trapezoid shape that allows bending of the fixing members 120, 121 without reducing the space between fixing members 120, 121 even if the distance between them would be reduced.

An optional fold line 186 may be used that may ease the bending of fixing members 120, 121 and/or of flexible connections 180, 182, 184. Connecting portions 130 may be made thinner at the kink line 186 along a continuous line or along of short lines that have distanced areas in between where the thickness of the surrounding area is maintained. Alternatively, a perforation may be applied, i.e. through holes may be used along kink line 186, for instance elongated or circular through holes.

The comparably short and compact retaining member body 150, i.e. whole container retaining member 100, may be inserted and/or fastened into an elongated container holder that defines the retaining space for the container or that surrounds it. Alternatively, container retaining member 100 may comprise a retaining member body 150 that has an elongated shape, see for instance Figures 3A to 4C.

Figure 2 illustrates the container retaining member 100 of Figure 1 in a second state. The second state may be a state in which a container is inserted into the container retaining member 100. However, the container is not shown in Figure 2 in order to not to obscure the relevant features. Alternatively, the state that is shown in Figure 2 may be reached by folding the fixing members 120, 121 and/or connections 180 to 182, for instance along fold line 186, using a special tool, for instance a mandrel having a cone shape. This intermediate step may ease the insertion of the container.

In the second state, first fixing portion 132 and second fixing portion 142 may protrude to the central axis of container retaining member 100 or of retaining member body 150 that are both arranged coaxially to each other. Thus, fixing portions 132 and 134 may hold and fix the container that is inserted into an insertion opening of retaining member body 150. This is explained in more detail for other embodiments below that are shown in Figure 3A to 4C.

Figure 2 also shows a film hinge 190 that connects first fixing member 120 and retaining member body 150. Film hinge 190 may have a thickness within the range that is stated in the first part of this document.

Figures 3A and 3B illustrate cross sections along an insertion axis A of a second embodiment of a container retaining member 300 having an elongated retaining member body 302. Container retaining member 300 may be in a first state in which no container is inserted. Alternatively, container retaining member 300 may be in a second state in which a container 310 is held within container retaining member 300. Container retaining member 300 and container 310 form a container retaining assembly 301 in the second state.

Container retaining member 300 comprises:
- a retaining member body 302 that may have a cylindrical shape, i.e. more precisely the shape of a hollow cylinder,
- an inner surface 304 of retaining member body 302,
- an outer surface 306 of retaining member body 302.

Inner surface 304 may surround or limit a retaining space 308 that may accommodate container 310. Retaining space 308 may have the same volume like container 310, a greater volume, for instance at most 10 percent greater, or a smaller volume, for instance at most 10 percent smaller.

Container 310 may comprise:
- a container body 312, for instance made of glass or comprising glass,
- a piston 314 at one end (proximal P) of container body 312, and
- a container cap 318 at the other end (distal D) of container body 312.

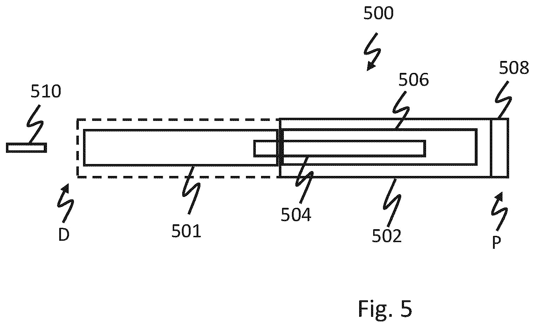

The container cap 318 may comprise for instance a metal or may be made of metal. The container cap 318 may have a through hole through which a needle may be inserted into the inside of container 310, see Figure 5.

Container 310 may be filled with a drug 316. The drug may comprise insulin, hormones, antibodies, one of the drugs listed on the following pages or another drug.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or may be used on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber container configured to store two or more components of the pharmaceutical formulation to-be-administered (e.g., an API and a diluent, or two different drugs) separately, one in each chamber. In such instances, the two chambers of the dual-chamber container may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (antidiabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codeable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An examples of an oligonucleotide is, for example: mipomersen sodium (Kynamro^{®}), a cholesterol-reducing antisense therapeutic for the treatment of familial hypercholesterolemia.

Examples of DPP4 inhibitors are Vildagliptin, Sitagliptin, Denagliptin, Saxagliptin, and Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Furthermore with regard to Figure 3A, container retaining member 300 may comprise an optional distal stop element 319 that may be in physical contact with a distal face of container cap 318 in the second state. A distal end D of container retaining member 300 is the end that is close to container cap 318. Furthermore, an insertion axis A is shown that extends along the longitudinal axis of container retaining member 300 and also along the longitudinal axis of retaining member body 302. A radial direction R is directed radially outwardly from insertion axis A. Container 310 is inserted along insertion axis A into retaining space 308.

Fixing members 320, 321 etc. are arranged in the distal part of container retaining member 300. An area 322 around fixing member 320 is shown in Figure 3B in more detail, i.e. magnified.

Still referring to Figure 3A, there is a first portion 370 of retaining space 308. First portion 370 is located between optional distal stop element 319 and fixing members 320, 321. A second portion 372 of retaining space 308 is located between fixing members 320, 321 and the proximal end P of container retaining member 300. First portion 370 is much smaller than second portion 372. The first part of this documents specifies examples for the volume of second portion 372 if compared with the overall volume of retaining space 308. Fixing members 320, 321 may be formed integrally with retaining member body 302.

However, it is also possible to place fixing members 320, 321 in the middle portion of retaining member body 302 or at the proximal end of retaining member body 302. In both cases, the container 310 may be modified, e.g. the container may have notches or recesses on respective positions that correspond to the position of the fixing members.

Figure 3B shows a magnification of the area 322 shown in Figure 3A. First fixing member 320 may comprise in this sequence:
- a connecting portion 330,
- a first fixing portion 332, and
- a second fixing portion 342.

Connecting portion 330 may be connected to retaining member body 302. First fixing portion 332 may be connected to connecting portion 330 and may further comprise a first free end 334.

Second fixing portion 342 may be connected only to first fixing portion 332 and may comprise a second free end 344.

A first planar surface SF1a may be formed on first fixing portion 332 and may face into the distal direction in the second state. In the second state, surface SF1a may be perpendicular relative to insertion axis A. Surface SF1a may also have another inclination relative to the insertion axis A as described in the first part of this document. Alternatively, surface SF1a may be a concave surface or a convex surface.

A second planar surface SF2a may be formed on second fixing portion 342 and may face into a direction that is opposite to the radial direction R in the second state. In the second state, surface SF2a may be parallel relative to insertion axis A. Surface SF2a may also have another inclination relative to the insertion axis A as described in the first part of this document. Alternatively, surface SF2a may be a concave surface or a convex surface. Second fixing member 342 may have a constant thickness. Alternatively other shapes are possible.

The angle between surfaces SF1a and SF2a is 90 degree in the example. However, other angles may be useful as well. The first part of the document specifies a range for this angle too, especially a range for an acute angle.

A third planar surface SF3a may be formed on first fixing portion 332 and may face proximally in the second state. In the second state, surface SF3a may be inclined relative to insertion axis A. Surface SF3a may also have another inclination compared with the inclination shown in Figure 3B relative to the insertion axis A. This is also described in the first part of this document in more detail. Alternatively, surface SF3a may be a concave surface or a convex surface. Third planar surface SF3a may be used to guide the cap or head of container 310 into an insertion opening 354 that is formed by fixing members 320, 321 etc.

There may be an optional free space 352 or clearance between first fixing portion 332 and retaining member body 302. Alternatively, the first fixing portion 332 may contact the retaining member body 302, i.e. without forming a free space 352 (clearance).

In an alternative embodiment, retaining member body 350 may be used instead of retaining member body 302. Retaining member body 302 may be replaced by a cylindrical container holder in this alternative embodiment. The first embodiment may be used, i.e. container retaining member 100 that comprises retaining member body 150.

No axial movement of container cap 318 and of container 310 is possible any more, i.e. neither distally D nor proximately P. It is not possible to remove container 310, especially container cap 318, out of container retaining member 400 or out of retaining member body 302 without destroying container 310 and/or some or all of fixing members 320, 321 etc. Mechanical access to fixing members 320, 321 etc. is hindered or impossible because fixing members 320, 321 are enclosed from all sides by respective parts of container retaining member 300 and or of container 310.

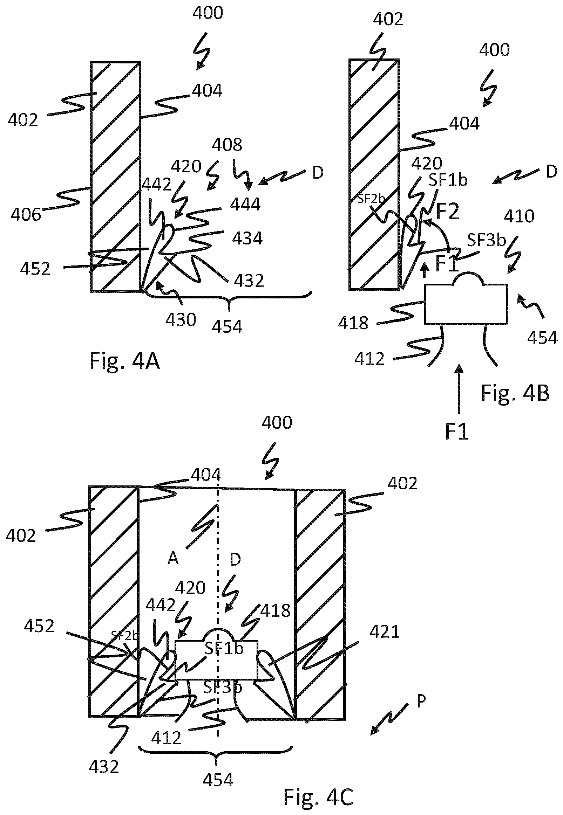

Figures 4A, 4B and 4C illustrate cross sections along an insertion axis A of a third embodiment of a container retaining member 400 and more specifically the steps of the insertion of a container into container retaining member 400. Figure 4A shows a first state in which no container 410 is placed in a retaining space 408. Figure 4B shows the insertion of container 410. Figure 4C shows a second state in which container 410 is placed and fixed within container retaining member 400.

Container retaining member 400 may comprise an elongated and preferably cylindrical retaining member body 402. Retaining member body 402 may comprise:
- an inner surface 404, and
- an outer surface 406.

Inner surface 404 may surround or limit a retaining space 408 that may retain the container 410 and/or a needle 510, see Figure 5. Container 410 may comprise a cylindrical container body 412 made for instance made of glass and a container cap 418 that may comprise for instance border crimped metal. Container 410 may be filled with the same kind of drug as container 310, see the drug list that is mentioned above.

Within container retaining member 400 and on retaining member body 402 there is a plurality of fixing members, see for instance fixing member 420 and fixing member 421 that is only shown in Figure 4C. First fixing member 420 may comprise:
- a connecting portion 430,
- a first fixing portion 432, and
- a second fixing portion 442.

The other fixing members, for instance 421, may be formed in the same way as fixing member 420, i.e. all fixing members 420, 421 etc. may have the same shape. Connecting portion 430 may be connected to retaining member body 402. First fixing portion 432 may be connected to connecting portion 430 and may further comprise a first free end 434. Second fixing portion 442 may also be connected to the connecting portion 430 and/or to first fixing portion 432 and may comprise a second free end 444. Second fixing portion 442 is arranged more distally compared to first fixing portion 432 in the first state and in the second state.

Between first fixing portion 432 and second fixing portion 442 there is a clearance in which a part of container cap 418 of the container 410 may be retained, see Figure 4C, i.e. both fixing portions 432 and 442 abut against the container 410 in the second state. Container cap 418 is arranged between first free end 434 and second free end 444. One of the free ends 434 and 444 or both free ends 434 and 444 may abut container cap 418 in the second state, i.e. may make physical contact.

In the first state, there may be a free space 452 (clearance) between pivotable fixing member 420 and inner surface 404. Alternatively, fixing member 430 may have a different inclination relative to inner surface 404 than shown in Figure 4A in the first state.

Figures 4A and 4C show an insertion opening 454 that is formed by the fixing members 420, 421 etc. Container 410 is inserted into insertion opening 454 along an insertion axis A, see figure 4C.

Figure 4B shows an intermediate state between first state and second state. There are the following surfaces on fixing member 420:
- a first surface SF1b on first fixing portion 432,
- a second surface SF2b on second fixing portion 442, and
- a third surface SF3b on first fixing portion 432.

Container 410 is inserted through the insertion opening 454 thereby guiding along the third surface SF3. A force F1 is applied to container 410 on insertion. Insertion may be done manually or automatically. A part of force F1 is used to pivot fixing member 420 to inner surface 504, i.e. to move the first fixing portion 432 and the second fixing portion 442 axially and outwardly. Thereby a force F2 is applied to fixing member 420. The same pivoting happens to the other fixing members 421, etc. that form insertion opening 454. Free space 452 may be used to retain fixing member 420 during the insertion of container cap 418 into insertion opening 454. This means that fixing member 420 may contact inner surface 404, especially the second fixing portion 442 and/or a side of the fixing member 420 that faces to inner surface 404. Alternatively, no contact is made between fixing member 420 and inner surface 404 during the insertion of container 410.

As shown in Figure 4C, first fixing portion 432 snaps behind cap 418 when the container cap 418 is inserted further along insertion axis A distally. At the same moment, second fixing portion 442 also abuts against container cap 418. Thus both fixing portions 432 and 442 hold a part of container cap 418 and fix it in its position. Both fixing portions 432 and 442 may prevent each other from losing contact to container 410 or more specifically to container cap 418. No axial movement of container cap 418 and of container 410 is possible any more, i.e. neither distally D nor proximately P. It is not possible to remove container 410, especially container cap 418, out of container retaining member 400 or out of retaining member body 402 without destroying container 410 and/or some or all of fixing members 420, 421 etc. Mechanical access to fixing members 420, 421 etc. is hindered or impossible because they are enclosed laterally and proximally by respective parts of container retaining member 400 and/or of container 410.

First surface SF1b may be a planar surface SF1b that may face into the distal direction in the second state. In the second state, surface SF1b may be perpendicular relative to insertion axis A. Surface SF1b may also have another inclination relative to the insertion axis A as shown in Figure 4C and as mentioned in the first part of this document. Alternatively, surface SF1b may be a concave surface or a convex surface.

Second surface SF2b may be a planar surface SF2b and may face into a direction that is opposite to the radial direction R in the second state. In the second state, surface SF2b may be parallel relative to insertion axis A. Surface SF2b may also have another inclination relative to the insertion axis A as shown in Figure 4C and as described in the first part of this document. Alternatively, surface SF2b may be a concave surface or a convex surface. Second fixing member 342 may have a thickness that varies depending on the distance to second free end 442. Alternatively other shapes are possible.

The angle between surfaces SF1b and SF2b may be 90 degree. However, other angles may be useful as well, especially acute angles as shown in Figure 4C. The first part of the document specifies a range for this angle too.

Third surface SF3b may be a planar surface SF3a and may face proximally in the second state. In the second state, surface SF3b may be inclined relative to insertion axis A as shown in Figure 4C. Surface SF3b may also have another inclination relative to the insertion axis A compared with the inclination shown in Figure 4C. This is also described in the first part of this document in more detail. Alternatively, surface SF3b may be a concave surface or a convex surface.

Optional free space 452 or clearance between fixing member 420 and retaining member body 402 may again have a greater volume compared to the intermediate state shown in Figure 4B. Alternatively, fixing member 420 may contact the inner surface 404 of retaining member body 402 in the second state, i.e. without forming a free space 452 (clearance). The material of fixing member 420 and/or of retaining member body 402 may have an appropriate flexibility and/or resiliency in this case without free space 352.

Figure 5 illustrates a drug delivery device 500 that comprises a container retaining member 501 according to one of the embodiments shown in Figure 1 to Figure 4C. The drug delivery device 500 may comprise a main housing part 502 that houses the container retaining member 501 completely or partially and that comprises further parts of the drug delivery device 500. Alternatively the main housing part 502 may be connected to the container retaining member 501 but may not surround it and even may not surround a part of the container retaining member 501, see dashed line in Figure 5.

Within the main housing part 502 the following may be arranged:
- a piston rod 504 that is adapted to move the piston of the container that is within container retaining member 501,
- a driving mechanism 506 for the piston rod 504. The driving mechanism 506 may comprise an energy storing element, for instance a spring, that is loaded manually or automatically, for instance during assembling of drug delivery device 500,
- for instance at an proximal end P, an actuating element 508 that is used for the initiation of a movement of the piston rod 504 into the container retaining member 501, whereby the driving mechanism 506 is used.

Drug delivery device 500 may be a single use or a multiple use device. Actuating element 508 may be part of a trigger mechanism that is triggered from the distal end, for instance if drug delivery device 500 is an auto injecting device.

The drug may be dispensed from the container through a needle 510 or a nozzle that is connectable and/or connected to the distal end D of the drug delivery device 500. The drug delivery device 500 may be a single use device or a multiple use device. The needle 510 may be changed before each use.

Spoken with other words a mechanism has been described for the fixation of a primary packing means, for instance a container or an ampoule.

Flexible latches (fixing members) may be embedded in the container holder (container retaining member) during primary shaping/molding. The flexible latch assembly may consist of multiple latches arranged on the inside of the container holder in a circular manner around the center of the container holder in the front section, e.g. where the needle interface is. These latches may have a certain shape so they provide a counter force during container assembly in order to be able to achieve a controlled assembly process. In the natural state (first state), these latches may have a certain clearance to the container holder's inner surface, so they can flex towards the inner surface to allow for the container to pass by so it can be placed in the correct position within the container holder during assembly (see for instance Figures 4A to 4C). The latches may be designed in such way that once the final position of the container within the container holder is reached, i.e. the crimp cap/top section of the container has passed the latch assembly, the latches may flip into the "lock" position by means of the material-inherent restoring force, see for instance Figure 3B. A material-inherent restoring force is optional in the example that is shown in Figures 4A to 4C. Another word for "material-inherent restoring force" is "resiliency or elasticity. In this "lock" position, it may be only possible to remove the container by excessive force which leads to either deformation/breakage of the latches, or more likely but dependent on the detail design of the latches disintegration of the container. The details of the design may be thickness, latch/holder material properties and so on. This fixation mechanism may be considered "tamper proof" as it is not possible to remove the container without destruction of either latches or container. These flexible latches may also be produced as separate assemblies and may be assembled into the container holder or on the container. In this case, the container holder may be designed in a way to allow for arresting the flexible latch assembly in the required position, e.g. by means of a geometrical feature within the container holder. Alternatively, chemical fixation, for instance glue, may be used to combine container holder and flexible latch assembly.

For some applications containers may need to be fixed in a container holder so that they cannot be removed from it by usual means. Other concepts for container fixation may rely on the dimension and shape of the crimp cap and may show some weaknesses - namely the removal force level variation and the unclear status of the container cap after mechanical deformation during assembly. The dimension and shape of the crimp cap may be hard to control.

The proposed container retaining member may solve the above mentioned problems by using a flexible, yet powerful connection technique - flexible latches or flexible fixing members. A flexible member within the container holder may be used to hold the container in the neck section underneath the crimp cap. It is therefore rather independent of the crimp cap dimensions, for instance diameter and/or concentricity.

Although embodiments of the present disclosure and their advantages have been described in detail, it should be understood that various changes, substitutions and alterations can be made therein without departing from the scope of the disclosure as defined by the appended claims. For example, it will be readily understood by those skilled in the art that many of the features, functions, processes and methods described herein may be varied while remaining within the scope of the present disclosure. Moreover, the scope of the present application is not intended to be limited to the particular embodiments of the system, process, manufacture, method or steps described in the present disclosure. Accordingly, the appended claims are intended to include within their scope such systems, processes, methods or steps. Further, it is possible to combine embodiments mentioned in the first part of the description with examples of the second part of the description which relates to Figures 1 to 5.

### List of reference signs

- A: insertion axis
- D: distal end
- P: proximal end
- R: radial direction
- 100: container retaining member
- 120, 121: fixing member
- 130: first connecting portion
- 132: first fixing portion
- 142: second fixing portion
- 150: retaining member body
- 180, 182, 184: flexible connection
- 186: fold line
- 190: film hinge
- SF1a, SF1b: first surface
- SF2a, SF2b: second surface
- SF3a, SF3b: third surface
- 300: container retaining member
- 301: assembly
- 302: retaining member body
- 304: inner surface
- 306: outer surface
- 308: retaining space
- 310: container
- 312: container body
- 314: piston
- 316: drug
- 318: container cap
- 319: distal stop element
- 320, 321: fixing member
- 322: area
- 330: connecting portion
- 332: first fixing portion
- 334: first free end
- 342: second fixing portion
- 344: second free end
- 350: retaining member body
- 352: free space
- 354: insertion opening
- 370: first portion
- 372: second portion
- 400: container retaining member
- 402: retaining member body
- 404: inner surface
- 406: outer surface
- 408: retaining space
- 410: container
- 412: container body
- 418: container cap
- 420, 421: fixing member
- 430: connecting portion
- 432: first fixing portion
- 434: first free end
- 442: second fixing portion
- 444: second free end
- 452: free space
- 454: insertion opening
- F1, F2: force
- 500: drug delivery device
- 501: container retaining member
- 502: main housing part
- 504: piston rod
- 506: driving mechanism
- 508: actuating element
- 510: needle

## Claims

1. Container retaining member (300) comprising:
a retaining member body (302) comprising an inner surface (304) that limits a retaining space (308) for a container (310), wherein the retaining member body (302) has an insertion opening (354) through which the container (310) is insertable into the retaining space (308), and
a fixing member (320) comprising:
a connecting portion (330) that is connected to the inner surface (304) and extends inwardly from the inner surface (304) of the retaining member body (302),
a first fixing portion (332) that extends from the connecting portion (330) into the retaining space (308) and comprises a first free end (334),
a second fixing portion (342) that extends from the connecting portion (330) and/or from the first fixing portion (332) into the retaining space (308) and comprises a second free end (344),
wherein the first fixing portion (332) and the second fixing portion (342) are arranged and configured to engage the container (310) in order to secure the container (310) within the container retaining member (300),
wherein the first free end (334) is arranged closer to the insertion opening (354) than the second free end (344),
wherein the connecting portion (330) is movably connected to the inner surface (304) of the retaining member body (302),
wherein the fixing member (320) is configured such that for switching between a first state and a second state the first free end (334) and/or the second free end (344) move(s) axially relative to an insertion axis (A) that is arranged crosswise or perpendicular to the insertion opening (454),
wherein in the first state no container (310) is inserted into the container retaining member (300),
wherein in the second state the container (310) is held within the container retaining member (300),
wherein the insertion opening (354) is widened by this movement in order to allow a cap (318) of the container (310) to pass,
wherein the axial movement is possible because the fixing member (320) pivots within the retaining member body (302) around a fixing position on the retaining member body (302),
wherein in the second state, the first free end (334) is arranged within a neck portion of the container (310), and
wherein in the second state, the second free end (344) is arranged laterally to the cap (318) and in contact with the cap (318).

2. Container retaining member (300) according to claim 1, wherein the fixing member (320) is configured such that for the switching between the first state and the second state the first free end (334) and/or the second free end (344) move(s) away from the insertion opening (454).

3. Container retaining member (300) according to claim 2, wherein the fixing member (320) is configured such that in the second state the second fixing portion (344) extends axially relative to the insertion axis (A) and/or that in the second state the second fixing portion (344) is arranged at a radial position relative to the insertion axis (A), wherein this radial position is radially inwardly offset from the inner surface (304) of the retaining member body (302).

4. Container retaining member (300) according to claim 2 or 3, wherein the fixing member (320) is configured such that in the second state the second fixing portion (344) is arranged between the container (310) and the inner surface (304) of the retaining member body (302).

5. Container retaining member (300) according to one of the preceding claims, wherein a flexible portion is arranged between the inner surface (304) of the retaining member body (302) and the connecting portion (330).

6. Container retaining member (300) according to claim 5, wherein the flexible portion comprises a film hinge (190),
and/or wherein at least one lateral flexible connection (182) is arranged between the first connecting portion of the first fixing member (120) and a second connecting portion of a second fixing member (121).

7. Container retaining member (300) according to one of the preceding claims, wherein the retaining member body (302) is an elongated retaining member body (302), and
wherein the fixing member (320) is formed unitarily with the elongated retaining member body (302) or wherein the fixing member (120) is formed on a separate part that is connected to the elongated retaining member body (302).

8. Container retaining member (100) according to one of the claims 1 to 6, wherein the retaining member body (150) has a length that is shorter than the width of the retaining member body (150) or shorter than the maximum outer diameter of the retaining member body (150) and the fixing member (120, 121) is formed unitarily with the retaining member body (150).

9. Container retaining member (300) according to any one of the preceding claims as far as referred back to claim 2,
wherein the first fixing portion (322) comprises a first surface (SF1a) that faces in the second state axially and/or distally relative to the insertion axis (A).

10. Container retaining member (300) according to any one of the preceding claims as far as referred back to claim 2,
wherein the second fixing portion (344) comprises a second surface (SF2a) that in the second state faces radially and/or inwardly relative to the insertion axis (A).

11. Container retaining member (300) according to claims 9 and 10, wherein the first surface (SF1a) and the second surface (SF2a) include an acute angle, preferably an angle that is in the range of 30 degrees to 85 degrees or in the range of 45 degrees to 75 degrees.

12. Container retaining member (300) according to any one of the preceding claims as far as referred to claim 2,
wherein the first fixing portion (332) comprises a third surface (SF3a) that is configured to guide the container (310) during the insertion of the container (310) into the retaining space (308),
and/or wherein in the second state the third surface (SF3a) faces into a direction that is different from the direction in which the first surface (SF1a) faces and/or that is different from the direction in which the second surface (SF2a) faces,
and/or wherein in the second state the third surface (SF3a) of the fixing member (320) is located closer to the insertion opening (354) than the first surface (SF1a).

13. Container retaining member (300) according to any one of the preceding claims, wherein the container retaining member (300) comprises three fixing members or at least four fixing members.

14. Drug delivery device (500) comprising a container retaining member (300) according to any one of the preceding claims and a container (310) comprising a drug (316), wherein the container (310) is connected to the container retaining member (300), and
wherein the fixing member (320) is in the second state that is different from the first state in which no container (310) is connected to the container retaining member (300),
the drug delivery device (500) further comprising a main housing part (502) that is connected to the assembly (301), and
a drive mechanism (506) being retained in the main housing part (502), wherein the drive mechanism (506) is operable to dispense drug (316) from the container (310).

15. Method for using a container retaining member (300) according to any one of the claims 1 to 13, comprising:
a) providing the container retaining member (300),
b) inserting the container (310) through the insertion opening (354) into the retaining space (308),
c) moving or pivoting at least a part of the fixing member (320) or the whole fixing member (320) when the container (310) abuts the fixing member (320) during the insertion of the container (310),
whereby at least a part of the fixing member (320) is moved from a first state to a second state by moving the first free end (334) and/or the second free end (344) away from the insertion opening (354) and axially relative to an insertion axis (A) that is arranged crosswise or perpendicular to the insertion opening (354).

## Patentansprüche

1. Behälterhalteelement (300), umfassend:
einen Halteelementkörper (302), der eine Innenfläche (304) umfasst, die einen Halteraum (308) für einen Behälter (310) begrenzt, wobei der Halteelementkörper (302) eine Einführungsöffnung (354) aufweist, durch die der Behälter (310) in den Halteraum (308) einführbar ist, und
ein Befestigungselement (320), das Folgendes umfasst:
einen Verbindungsabschnitt (330), der mit der Innenfläche (304) verbunden ist und sich von der Innenfläche (304) des Halteelementkörpers (302) nach innen erstreckt,
einen ersten Befestigungsabschnitt (332), der sich von dem Verbindungsabschnitt (330) in den Halteraum (308) erstreckt und ein erstes freies Ende (334) umfasst,
einen zweiten Befestigungsabschnitt (342), der sich von dem Verbindungsabschnitt (330) und/oder von dem ersten Befestigungsabschnitt (332) in den Halteraum (308) erstreckt und ein zweites freies Ende (344) umfasst,
wobei der erste Befestigungsabschnitt (332) und der zweite Befestigungsabschnitt (342) dazu angeordnet und ausgestaltet sind, den Behälter (310) in Eingriff zu nehmen, um den Behälter (310) in dem Behälterhalteelement (300) zu befestigen,
wobei das erste freie Ende (334) näher an der Einführungsöffnung (354) als das zweite freie Ende (344) angeordnet ist,
wobei der Verbindungsabschnitt (330) beweglich mit der Innenfläche (304) des Halteelementkörpers (302) verbunden ist,
wobei das Befestigungselement (320) so ausgestaltet ist, dass sich das erste freie Ende (334) und/oder das zweite freie Ende (344) zum Umschalten zwischen einem ersten Zustand und einem zweiten Zustand axial relativ zu einer Einführungsachse (A) bewegen, die quer oder senkrecht zu der Einführungsöffnung (454) angeordnet ist,
wobei in dem ersten Zustand kein Behälter (310) in das Behälterhalteelement (300) eingeführt ist,
wobei in dem zweiten Zustand der Behälter (310) in dem Behälterhalteelement (300) gehalten wird,
wobei die Einführungsöffnung (354) durch diese Bewegung erweitert wird, damit eine Kappe (318) des Behälters (310) hindurchgehen kann,
wobei die axiale Bewegung möglich ist, weil das Befestigungselement (320) in dem Halteelementkörper (302) um eine Befestigungsposition an dem Halteelementkörper (302) schwenkt,
wobei in dem zweiten Zustand das erste freie Ende (334) in einem Halsabschnitt des Behälters (310) angeordnet ist und
wobei in dem zweiten Zustand das zweite freie Ende (344) lateral zu der Kappe (318) und in Kontakt mit der Kappe (318) angeordnet ist.

2. Behälterhalteelement (300) nach Anspruch 1, wobei das Befestigungselement (320) so ausgestaltet ist, dass sich das erste freie Ende (334) und/oder das zweite freie Ende (344) zum Umschalten zwischen dem ersten Zustand und dem zweiten Zustand von der Einführungsöffnung (454) weg bewegen.

3. Behälterhalteelement (300) nach Anspruch 2, wobei das Befestigungselement (320) so ausgestaltet ist, dass sich der zweite Befestigungsabschnitt (344) im zweiten Zustand axial relativ zu der Einführungsachse (A) erstreckt und/oder dass der zweite Befestigungsabschnitt (344) im zweiten Zustand in einer radialen Position relativ zu der Einführungsachse (A) angeordnet ist, wobei diese radiale Position von der Innenfläche (304) des Halteelementkörpers (302) radial nach innen versetzt ist.

4. Behälterhalteelement (300) nach Anspruch 2 oder 3, wobei das Befestigungselement (320) so ausgestaltet ist, dass der zweite Befestigungsabschnitt (344) im zweiten Zustand zwischen dem Behälter (310) und der Innenfläche (304) des Halteelementkörpers (302) angeordnet ist.

5. Behälterhalteelement (300) nach einem der vorhergehenden Ansprüche, wobei ein flexibler Abschnitt zwischen der Innenfläche (304) des Halteelementkörpers (302) und dem Verbindungsabschnitt (330) angeordnet ist.

6. Behälterhalteelement (300) nach Anspruch 5, wobei der flexible Abschnitt ein Filmscharnier (190) umfasst,
und/oder wobei mindestens eine seitliche flexible Verbindung (182) zwischen dem ersten Verbindungsabschnitt des ersten Befestigungselements (120) und einem zweiten Verbindungsabschnitt eines zweiten Befestigungselements (121) angeordnet ist.

7. Behälterhalteelement (300) nach einem der vorhergehenden Ansprüche, wobei der Halteelementkörper (302) ein längserstreckter Halteelementkörper (302) ist und
wobei das Befestigungselement (320) einteilig mit dem längserstreckten Halteelementkörper (302) ausgebildet ist oder wobei das Befestigungselement (120) an einem mit dem längserstreckten Halteelementkörper (302) verbundenen separaten Teil ausgebildet ist.

8. Behälterhalteelement (100) nach einem der Ansprüche 1 bis 6, wobei der Halteelementkörper (150) eine Länge aufweist, die kürzer als die Breite des Halteelementkörpers (150) oder kürzer als der maximale Außendurchmesser des Halteelementkörpers (150) ist, und das Befestigungselement (120, 121) einteilig mit dem Halteelementkörper (150) ausgebildet ist.

9. Behälterhalteelement (300) nach einem der vorhergehenden Ansprüche, soweit auf Anspruch 2 rückbezogen,
wobei der erste Befestigungsabschnitt (322) eine erste Fläche (SF1a) umfasst, die in dem zweiten Zustand axial und/oder distal relativ zu der Einführungsachse (A) weist ist.

10. Behälterhalteelement (300) nach einem der vorhergehenden Ansprüche, soweit auf Anspruch 2 rückbezogen,
wobei der zweite Befestigungsabschnitt (344) eine zweite Fläche (SF2a) umfasst, die in dem zweiten Zustand radial und/oder nach innen relativ zu der Einführungsachse (A) weist ist.

11. Behälterhalteelement (300) nach Ansprüchen 9 und 10, wobei die erste Fläche (SF1a) und die zweite Fläche (SF2a) einen spitzen Winkel, vorzugsweise einen Winkel, der im Bereich von 30 Grad bis 85 Grad oder im Bereich von 45 Grad bis 75 Grad liegt, einschließen.

12. Behälterhalteelement (300) nach einem der vorhergehenden Ansprüche, soweit auf Anspruch 2 rückbezogen,
wobei der erste Befestigungsabschnitt (332) eine dritte Fläche (SF3a) umfasst, die dazu ausgestaltet ist, den Behälter (310) während des Einführens des Behälters (310) in den Halteraum (308) zu führen,
und/oder wobei die dritte Fläche (SF3a) in dem zweiten Zustand in eine Richtung weist, die sich von der Richtung unterscheidet, in die die erste Fläche (SF1a) weist, und/oder die sich von der Richtung unterscheidet, in die die zweite Fläche (SF2a) weist,
und/oder wobei sich die dritte Fläche (SF3a) des Befestigungselements (320) im zweiten Zustand näher an der Einführungsöffnung (354) als die erste Fläche (SF1a) befindet.

13. Behälterhalteelement (300) nach einem der vorhergehenden Ansprüche, wobei das Behälterhalteelement (300) drei Befestigungselemente oder mindestens vier Befestigungselemente umfasst.

14. Medikamenten-Verabreichungsvorrichtung (500), umfassend ein Behälterhalteelement (300) nach einem der vorhergehenden Ansprüche und einen Behälter (310), der ein Medikament (316) umfasst, wobei der Behälter (310) mit dem Behälterhalteelement (300) verbunden ist und
wobei sich das Befestigungselement (320) in dem zweiten Zustand befindet, der sich von dem ersten Zustand unterscheidet, in dem kein Behälter (310) mit dem Behälterhalteelement (300) verbunden ist,
wobei die Medikamenten-Verabreichungsvorrichtung (500) ferner ein Hauptgehäuseteil (502) umfasst, das mit der Baugruppe (301) verbunden ist, und
einen Antriebsmechanismus (506), der in dem Hauptgehäuseteil (502) gehalten ist, wobei der Antriebsmechanismus (506) zur Abgabe von Medikament (316) aus dem Behälter (310) betätigbar ist.

15. Verfahren zur Verwendung eines Behälterhalteelements (300) nach einem der Ansprüche 1 bis 13, umfassend:
a) Bereitstellen des Behälterhalteelements (300),
b) Einführen des Behälters (310) durch die Einführungsöffnung (354) in den Halteraum (308),
c) Bewegen oder Schwenken mindestens eines Teils des Befestigungselements (320) oder des gesamten Befestigungselements (320), wenn der Behälter (310) während des Einführens des Behälters (310) an dem Befestigungselement (320) anliegt,
wodurch zumindest ein Teil des Befestigungselements (320) aus einem ersten Zustand in einen zweiten Zustand bewegt wird, indem das erste freie Ende (334) und/oder das zweite freie Ende (344) von der Einführungsöffnung (354) weg und axial relativ zu einer quer oder senkrecht zu der Einführungsöffnung (354) angeordneten Einführungsachse (A) bewegt werden.

## Revendications

1. Élément de retenue de récipient (300) comprenant :
un corps d'élément de retenue (302) comprenant une surface interne (304) qui limite un espace de retenue (308) pour un récipient (310), le corps d'élément de retenue (302) présentant une ouverture d'insertion (354) à travers laquelle le récipient (310) peut être inséré dans l'espace de retenue (308), et
un élément de fixation (320) comprenant :
une partie de liaison (330) qui est reliée à la surface intérieure (304) et s'étend vers l'intérieur à partir de la surface intérieure (304) du corps d'élément de retenue (302),
une première partie de fixation (332) qui s'étend depuis la partie de liaison (330) dans l'espace de retenue (308) et comprend une première extrémité libre (334),
une seconde partie de fixation (342) qui s'étend depuis la partie de liaison (330) et/ou depuis la première partie de fixation (332) dans l'espace de retenue (308) et comprend une seconde extrémité libre (344),
la première partie de fixation (332) et la seconde partie de fixation (342) étant disposées et conçues pour venir en prise avec le récipient (310) afin de fixer le récipient (310) à l'intérieur de l'élément de retenue de récipient (300),
la première extrémité libre (334) étant disposée plus près de l'ouverture d'insertion (354) que la seconde extrémité libre (344),
la partie de liaison (330) étant reliée mobile à la surface intérieure (304) du corps d'élément de retenue (302),
l'élément de fixation (320) étant conçu de sorte que pour commuter entre un premier état et un second état, la première extrémité libre (334) et/ou la seconde extrémité libre (344) se déplacent axialement par rapport à un axe d'insertion (A) qui est disposé transversalement ou perpendiculairement à l'ouverture d'insertion (454),
dans le premier état, aucun récipient (310) n'étant inséré dans l'élément de retenue de récipient (300),
dans le second état, le récipient (310) étant maintenu à l'intérieur de l'élément de retenue de récipient (300),
l'ouverture d'insertion (354) étant élargie par ce mouvement pour laisser passer un bouchon (318) du récipient (310),
le mouvement axial étant possible parce que l'élément de fixation (320) pivote à l'intérieur du corps d'élément de retenue (302) autour d'une position de fixation sur le corps d'élément de retenue (302),
dans le second état, la première extrémité libre (334) étant disposée au sein d'une partie col du récipient (310), et
dans le second état, la seconde extrémité libre (344) étant disposée latéralement au bouchon (318) et au contact du bouchon (318).

2. Élément de retenue de récipient (300) selon la revendication 1, l'élément de fixation (320) étant conçu de sorte que, pour la commutation entre le premier état et le second état, la première extrémité libre (334) et/ou la seconde extrémité libre (344) s'éloignent de l'ouverture d'insertion (454).

3. Élément de retenue de récipient (300) selon la revendication 2, l'élément de fixation (320) étant conçu de sorte que, dans le second état, la seconde partie de fixation (344) s'étende axialement par rapport à l'axe d'insertion (A) et/ou que, dans le second état, la seconde partie de fixation (344) soit disposée au niveau d'une position radiale par rapport à l'axe d'insertion (A), cette position radiale étant décalée radialement vers l'intérieur par rapport à la surface intérieure (304) du corps d'élément de retenue (302).

4. Élément de retenue de récipient (300) selon la revendication 2 ou 3, l'élément de fixation (320) étant conçu de sorte que, dans le second état, la seconde partie de fixation (344) soit disposée entre le récipient (310) et la surface intérieure (304) du corps d'élément de retenue (302).

5. Élément de retenue de récipient (300) selon l'une des revendications précédentes, une partie flexible étant disposée entre la surface intérieure (304) du corps d'élément de retenue (302) et la partie de liaison (330).

6. Élément de retenue de récipient (300) selon la revendication 5, la partie flexible comprenant une charnière film (190),
et/ou au moins une liaison flexible latérale (182) étant disposée entre la première partie de liaison du premier élément de fixation (120) et une seconde partie de liaison d'un second élément de fixation (121).

7. Élément de retenue de récipient (300) selon l'une des revendications précédentes, le corps d'élément de retenue (302) étant un corps d'élément de retenue (302) allongé, et
l'élément de fixation (320) étant formé d'une seule pièce avec le corps d'élément de retenue (302) allongé ou l'élément de fixation (120) étant formé sur une pièce séparée qui est reliée au corps d'élément de retenue (302) allongé.

8. Élément de retenue de récipient (100) selon l'une des revendications 1 à 6, le corps d'élément de retenue (150) ayant une longueur qui est inférieure à la largeur du corps d'élément de retenue (150) ou inférieure au diamètre extérieur maximal du corps d'élément de retenue (150) et l'élément de fixation (120, 121) étant formé d'une seule pièce avec le corps d'élément de retenue (150).

9. Élément de retenue de récipient (300) selon l'une quelconque des revendications précédentes en référence à la revendication 2,
la première partie de fixation (322) comprenant une première surface (SF1a) qui est tournée dans le second état axialement et/ou distalement par rapport à l'axe d'insertion (A).

10. Élément de retenue de récipient (300) selon l'une quelconque des revendications précédentes en référence à la revendication 2,
la seconde partie de fixation (344) comprenant une deuxième surface (SF2a) qui, dans le second état, est tournée radialement et/ou vers l'intérieur par rapport à l'axe d'insertion (A).

11. Élément de retenue de récipient (300) selon les revendications 9 et 10, la première surface (SF1a) et la deuxième surface (SF2a) comprenant un angle aigu, de préférence un angle qui est dans la plage comprise entre 30 degrés et 85 degrés ou dans la plage comprise entre 45 degrés et 75 degrés.

12. Élément de retenue de récipient (300) selon l'une quelconque des revendications précédentes en référence à la revendication 2,
la première partie de fixation (332) comprenant une troisième surface (SF3a) qui est conçue pour guider le récipient (310) lors de l'insertion du récipient (310) dans l'espace de retenue (308),
et/ou, dans le second état, la troisième surface (SF3a) étant tournée vers une direction différente de la direction vers laquelle la première surface (SF1a) est tournée et/ou différente de la direction vers laquelle la deuxième surface (SF2a) est tournée,
et/ou, dans le second état, la troisième surface (SF3a) de l'élément de fixation (320) étant située plus près de l'ouverture d'insertion (354) que la première surface (SF1a).

13. Élément de retenue de récipient (300) selon l'une quelconque des revendications précédentes, l'élément de retenue de récipient (300) comprenant trois éléments de fixation ou au moins quatre éléments de fixation.

14. Dispositif d'administration de médicament (500) comprenant un élément de retenue de récipient (300) selon l'une quelconque des revendications précédentes et un récipient (310) comprenant un médicament (316), le récipient (310) étant relié à l'élément de retenue de récipient (300), et
l'élément de fixation (320) étant dans le second état qui est différent du premier état dans lequel aucun récipient (310) n'est relié à l'élément de retenue de récipient (300),
le dispositif d'administration de médicament (500) comprenant en outre une partie de boîtier principale (502) qui est reliée à l'ensemble (301), et
un mécanisme d'entraînement (506) étant retenu dans la partie de boîtier principale (502), le mécanisme d'entraînement (506) pouvant être actionné pour distribuer le médicament (316) à partir du récipient (310).

15. Procédé d'utilisation d'un élément de retenue de récipient (300) selon l'une quelconque des revendications 1 à 13, comprenant les étapes consistant à :
a) fournir l'élément de retenue de récipient (300),
b) insérer le récipient (310) à travers l'ouverture d'insertion (354) dans l'espace de retenue (308),
c) déplacer ou faire pivoter au moins une partie de l'élément de fixation (320) ou l'élément de fixation (320) en entier lorsque le récipient (310) bute contre l'élément de fixation (320) lors de l'insertion du récipient (310),
moyennant quoi au moins une partie de l'élément de fixation (320) passe d'un premier état à un second état en éloignant la première extrémité libre (334) et/ou la seconde extrémité libre (344) de l'ouverture d'insertion (354) et en les déplaçant axialement par rapport à un axe d'insertion (A) qui est disposé transversalement ou perpendiculairement à l'ouverture d'insertion (354).
